# EUROPEAN PATENT SPECIFICATION

(11) **EP 2 621 585 B1**
(45) Date of publication and mention of the grant of the patent: **06.09.2017**
(21) Application number: 11717436.7
(22) Date of filing: 22.04.2011
(51) Int. Cl.: A61N 1/368, A61N 1/372, A61N 1/36, A61N 1/365, A61N 1/37

(54) **PRIORITIZED PROGRAMMING OF MULTI-ELECTRODE PACING LEADS**
PRIORISIERTE PROGRAMMIERUNG VON SCHRITTMACHERKABELN MIT MEHREREN ELEKTRODEN
PROGRAMMATION PRIORITAIRE DE DÉRIVATIONS DE STIMULATION À ÉLECTRODES MULTIPLES

(30) Priority: 29.09.2010 US 893517
(43) Date of publication of application: 07.08.2013
(73) Proprietor: Medtronic, Inc., Minneapolis, MN 55342-5604 (US)
(72) Inventor: SCHOTZKO, Elizabeth, A., Blaine, MN 55449 (US); SCHELL, Jon, D., Shoreview, MN 55126 (US)
(74) Representative: Hughes, Andrea Michelle
(86) International application number: PCT/US2011/033564
(87) International publication number: WO 2012/044370

(56) References cited:
- US-A1- 2006 074 454
- US-A1- 2008 046 019
- US-A1- 2009 043 352
- US-A1- 2010 152 801
- US-B1- 7 792 585

## Description

### TECHNICAL FIELD

This disclosure relates to implantable medical devices, and more particularly, to implantable medical devices that deliver cardiac pacing.

### BACKGROUND

A wide variety of implantable medical devices for delivering a therapy or monitoring a physiologic condition have been clinically implanted or proposed for clinical implantation in patients. In some cases, implantable medical devices (IMD) deliver electrical stimulation therapy and/or monitor physiological signals via one or more electrodes or sensor elements, which may be included as part of one or more elongated implantable medical leads. Implantable medical leads may be configured to allow electrodes or sensors to be positioned at desired locations for sensing or delivery of stimulation. For example, electrodes or sensors may be carried at a distal portion of the lead. A proximal portion of the lead may be coupled to an implantable medical device housing, which may contain electronic circuitry such as stimulation generation and/or sensing circuitry.

For example, implantable medical devices, such as cardiac pacemakers or implantable cardioverter-defibrillators, provide therapeutic stimulation to the heart by delivering electrical therapy signals, such as pulses for pacing, or shocks for cardioversion or defibrillation, via electrodes of one or more implantable leads. In some cases, such an implantable medical device may sense for intrinsic depolarizations of the heart, and control the delivery of such signals to the heart based on the sensing. When an abnormal rhythm is detected, which may be bradycardia, tachycardia or fibrillation, an appropriate electrical signal or signals may be delivered to restore the normal rhythm. For example, in some cases, an implantable medical device may deliver pacing, cardioversion or defibrillation signals to the heart of the patient upon detecting ventricular tachycardia, and deliver defibrillation electrical signals to a patient's heart upon detecting ventricular fibrillation. Pacing signals typically have a lower energy than the cardioversion or defibrillation signals.

Patients with heart failure are, in some cases, treated with cardiac resynchronization therapy (CRT). CRT is a form of cardiac pacing. In some examples, CRT involves delivery of pacing pulses to both ventricles to synchronize their contraction. In other examples, CRT involves delivery of pacing pulses to one ventricle to synchronize its contraction with that of the other ventricular, such as pacing the left ventricle to synchronize its contraction with that of the right ventricle. CRT is one example of a variety of modes of cardiac pacing in which stimulation is delivered to one chamber or location at a time that is an interval before or after an event at another chamber or location. The event at the other chamber or location may be the delivery of a pacing pulse to the other chamber or location, or the detection of an intrinsic cardiac depolarization at the other chamber or location.

Various methods exist for detecting whether a pacing stimulus has captured the heart and determining capture thresholds. In some examples, a first pair of electrodes delivers a pacing pulse to a chamber, and the same or a different pair of electrodes detects an electrical signal, e.g., evoked response, in the chamber indicative of capture. In other examples, a device detects a mechanical contraction of the heart at the target site as evidence of capture of the heart by the pacing stimulus. In general, capture threshold determination or management involves delivery of pacing stimuli at incrementally increasing or decreasing magnitudes, e.g., voltage or current amplitudes or pulse widths, and identification of the magnitude at which capture or loss of capture occurs.

US 2010/0152801, US 2009/0043352 and US 2008/046019 teach optimized selection of vectors.

### SUMMARY

In general, this disclosure is directed to techniques for facilitating selection of a pacing vector from amongst a plurality of available pacing vectors. In accordance with certain techniques of this disclosure, a user, e.g., clinician, may select particular pacing vectors to test as well as the type and priority of various test criteria, e.g., CRT efficacy, capture thresholds, R-wave amplitudes, phrenic nerve stimulation amplitudes, and impedance. Once the vectors have been tested, a display may rank, or prioritize, the vectors based on measurements of the various test criteria.

Certain techniques of this disclosure may be useful in aiding clinicians quickly select a particular electrode on a multi-electrode lead, e.g., a quadripolar lead, for pacing. In addition, various techniques of this disclosure may be useful in aiding clinicians quickly select a particular band and a particular electrode on that band on a multipolar lead.

In one example, the description is directed to a method of facilitating selection of at least one vector from among a plurality of vectors for pacing a chamber of a heart. The method comprises presenting, by a computing device, a plurality of criteria by which each of the plurality of vectors may be prioritized, selecting, by a user, at least one criterion from among the plurality of criteria by which each of the plurality of vectors may be prioritized, for each of the plurality of vectors, measuring the at least one selected criterion, and automatically prioritizing, by the computing device, the plurality of vectors based on the measurement of the at least one selected criterion.

In another example, the disclosure is directed to a system that facilitates selection of at least one vector from among a plurality of vectors for pacing a first chamber of a heart. The system comprises an implantable medical device configured to deliver pacing stimuli to the heart, and a processor configured to control presentation of a plurality of criteria by which each of the plurality of vectors may be prioritized, receive a selection of at least one criterion from among the plurality of criteria by which each of the plurality of vectors may be prioritized, for each of the plurality of vectors, control measurement of the at least one selected criterion, and automatically prioritize the plurality of vectors based on the measurement of the at least one selected criterion.

In another example, the description is directed to a computer-readable storage medium comprising instructions that, when executed by a processor, cause the processor to present a plurality of criteria by which each of the plurality of vectors may be prioritized, control presentation of a plurality of criteria by which each of the plurality of vectors may be prioritized, receive a selection of at least one criterion from among the plurality of criteria by which each of the plurality of vectors may be prioritized, for each of the plurality of vectors, control measurement of the at least one selected criterion, and automatically prioritize the plurality of vectors based on the measurement of the at least one selected criterion.

### BRIEF DESCRIPTION OF DRAWINGS

FIG. 1 is a conceptual diagram illustrating an example system that may be used to provide therapy to and/or monitor a heart of a patient.
FIG. 2 is a conceptual diagram illustrating the example implantable medical device (IMD) and the leads of the system shown in FIG. 1 in greater detail.
FIG. 3 is a block diagram illustrating an example configuration of an implantable medical device.
FIG. 4 is a flow diagram illustrating an example method for assessing a plurality of vectors in accordance with this description.
FIG. 5 is a flow diagram illustrating another example method for assessing a plurality of vectors in accordance with this description.
FIG. 6 is a conceptual diagram illustrating one example of an implantable multipolar stimulation lead.
FIGS. 7A-7C are transverse cross-sections of example multipolar stimulation leads having two or more electrodes around the circumference of the lead.
FIG. 8 is a flow diagram illustrating another example method for assessing a plurality of vectors in accordance with this description.
FIG. 9 is a functional block diagram illustrating an example configuration of the programmer of FIG. 1.
FIG. 10 is a flow diagram illustrating another example method for assessing a plurality of vectors in accordance with this description.
FIG. 11 is a block diagram illustrating an example system that includes a server and one or more computing devices that are coupled to the IMD and the programmer shown in FIG. 1 via a network.

### DETAILED DESCRIPTION

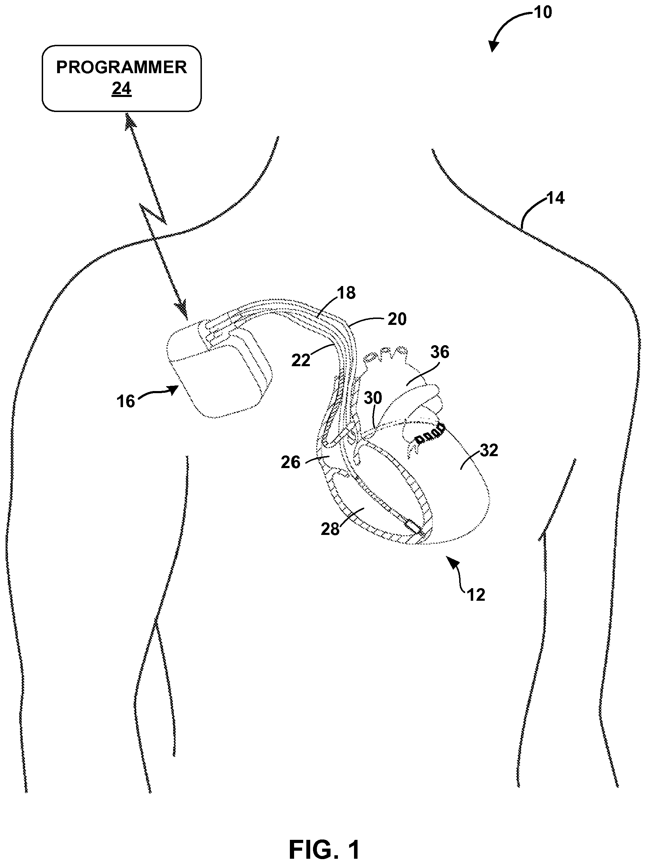

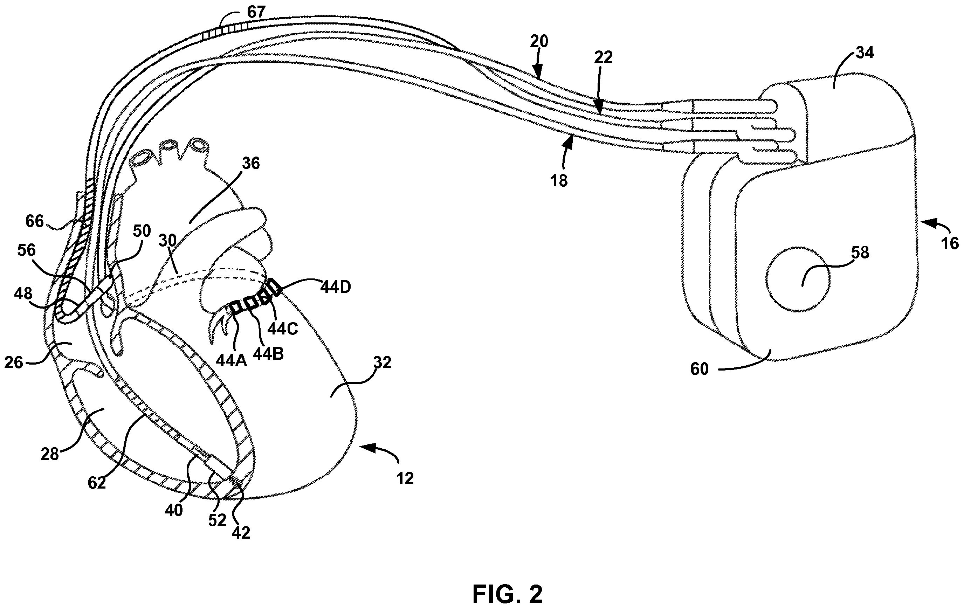

FIG. 1 is a conceptual diagram illustrating an example system 10 that may be used to monitor and/or provide therapy to heart 12 of patient 14. Patient 14 ordinarily, but not necessarily, will be a human. System 10 includes IMD 16, which is coupled to leads 18, 20, and 22, and programmer 24. IMD 16 may be, for example, an implantable pacemaker, cardioverter, and/or defibrillator that provides electrical signals to heart 12 via electrodes coupled to one or more of leads 18, 20, and 22. Two or more electrodes, and the polarity of the electrodes, define a vector, or path, for delivering pacing pulses to heart 12. In accordance with this disclosure, IMD 16 may deliver pacing pulses via a plurality of pacing vectors that may include at least one electrode on lead 20 in order to assess various criteria, e.g., CRT efficacy, capture thresholds, R-wave amplitudes, phrenic nerve stimulation amplitudes, impedance, and power source longevity, by which the pacing vectors may be prioritized, as will be described in greater detail below. In some examples, IMD 16 may deliver pacing pulses via a plurality of pacing vectors that may include at least one electrode on the housing of the IMD, e.g., housing electrode 58 (FIG. 2). IMD 16 may provide the measured criteria, data derived therefrom or alerts based thereon to programmer 24 via wireless telemetry.

Leads 18, 20, 22 extend into the heart 12 of patient 14 to sense electrical activity of heart 12 and/or deliver electrical stimulation to heart 12. In the example shown in FIG. 1, right ventricular (RV) lead 18 extends through one or more veins (not shown), the superior vena cava (not shown), and right atrium 26, and into right ventricle 28. Left ventricular (LV) coronary sinus lead 20 extends through one or more veins, the vena cava, right atrium 26, and into the coronary sinus 30 to a region adjacent to the free wall of left ventricle 32 of heart 12. Right atrial (RA) lead 22 extends through one or more veins and the vena cava, and into the right atrium 26 of heart 12.

IMD 16 may sense electrical signals attendant to the depolarization and repolarization of heart 12 via electrodes (not shown in FIG. 1) coupled to at least one of the leads 18, 20, 22. In some examples, IMD 16 provides pacing pulses to heart 12 based on the electrical signals sensed within heart 12. The configurations of electrodes used by IMD 16 for sensing and pacing may be unipolar or bipolar. IMD 16 may also provide defibrillation therapy and/or cardioversion therapy via electrodes located on at least one of the leads 18, 20, 22. IMD 16 may detect arrhythmia of heart 12, such as fibrillation of ventricles 28 and 32 or atrium 26, and deliver defibrillation therapy to heart 12 in the form of electrical pulses. In some examples, IMD 16 may be programmed to deliver a progression of therapies, e.g., pulses with increasing energy levels, until a fibrillation of heart 12 is stopped. IMD 16 detects fibrillation employing one or more fibrillation detection techniques known in the art.

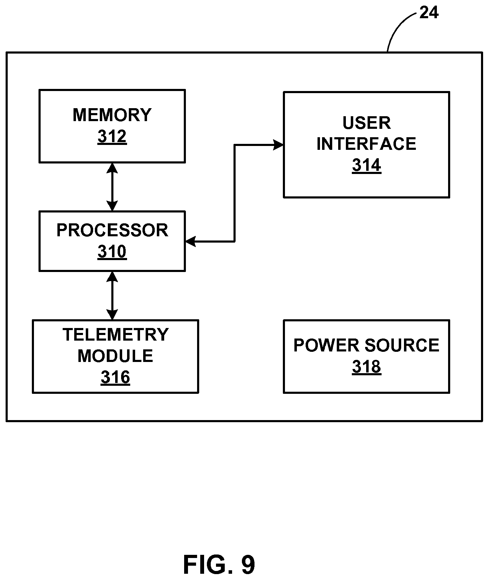

In some examples, programmer 24 (shown in greater detail in FIG. 9) may be a handheld computing device or a computer workstation. A user, such as a physician, technician, or other clinician, may interact with programmer 24 to communicate with IMD 16. For example, the user may interact with programmer 24 to retrieve physiological or diagnostic information from IMD 16. A user may also interact with programmer 24 to program IMD 16, e.g., select values for operational parameters of the IMD.

For example, the user may use programmer 24 to retrieve information from IMD 16 regarding the rhythm of heart 12, trends therein over time, or arrhythmic episodes. As another example, the user may use programmer 24 to retrieve information from IMD 16 regarding other sensed physiological parameters of patient 14, such as intracardiac or intravascular pressure, activity, posture, respiration, or thoracic impedance. As another example, the user may use programmer 24 to retrieve information from IMD 16 regarding the performance or integrity of IMD 16 or other components of system 10, such as leads 18, 20 and 22, or a power source of IMD 16. The user may use programmer 24 to program a therapy progression, select electrodes used to deliver defibrillation pulses, select waveforms for the defibrillation pulse, or select or configure a fibrillation detection algorithm for IMD 16. The user may also use programmer 24 to program aspects of other therapies provided by IMD 14, such as cardioversion or pacing therapies.

IMD 16 and programmer 24 may communicate via wireless communication using any techniques known in the art. Examples of communication techniques may include, for example, low frequency or radiofrequency (RF) telemetry, but other techniques are also contemplated. In some examples, programmer 24 may include a programming head that may be placed proximate to the patient's body near the IMD 16 implant site in order to improve the quality or security of communication between IMD 16 and programmer 24.

Using various techniques of this disclosure, a user may select various criteria, e.g., CRT efficacy, capture thresholds, R-wave amplitudes, phrenic nerve stimulation amplitudes, longevity, and impedance, by which a plurality of pacing vectors may be prioritized. IMD 16 may deliver pacing pulses via various combinations of electrodes that include at least one electrode on LV coronary sinus lead 20, for example. Subsequent to the delivery of each of the plurality of pacing pulses, another combination of electrodes that includes at least one electrode on RV lead 18 sense electrical activity in order to assess the various user-selected criteria, e.g., CRT efficacy, capture thresholds, R-wave amplitudes, phrenic nerve stimulation amplitudes, and impedance, by which the pacing vectors may be prioritized. IMD 16 automatically, i.e., without user intervention, prioritizes the plurality of pacing vectors based on the assessment of the criteria. That is, IMD 16 automatically ranks or orders the plurality of tested pacing vectors based on the assessment of the user-selected criteria. Then, IMD 16 presents the prioritized plurality of tested pacing vectors to the user, e.g., in a list of high to low ranking or low to high ranking, or the like, via a user interface, e.g., via a user interface on programmer 24.

FIG. 2 is a conceptual diagram illustrating IMD 16 and leads 18, 20, and 22 of therapy system 10 in greater detail. Leads 18, 20, 22 may be electrically coupled to a signal generator and a sensing module of IMD 16 via connector block 34. Each of the leads 18, 20, 22 includes an elongated insulative lead body carrying one or more conductors. Bipolar electrodes 40 and 42 are located adjacent to a distal end of lead 18 and bipolar electrodes 48 and 50 are located adjacent to a distal end of lead 22. In some example configurations, lead 20 may be a quadripolar lead and, as such, include four electrodes, namely electrodes 44A-44D, which are located adjacent to a distal end of lead 20. Electrodes 40, 44A-44D, and 48 may take the form of ring electrodes, and electrodes 42 and 50 may take the form of extendable helix tip electrodes mounted retractably within insulative electrode heads 52 and 56, respectively, or a passive lead with tines.

Leads 18 and 22 also include elongated intracardiac electrodes 62 and 66 respectively, which may take the form of a coil. In addition, one of leads 18, 20, 22, e.g., lead 22 as seen in FIG. 2, may include a superior vena cava (SVC) coil 67 for delivery of electrical stimulation, e.g., transvenous defibrillation. For example, lead 22 may be inserted through the superior vena cava and SVC coil 67 may be placed, for example, at the right atrial/SVC junction (low SVC) or in the left subclavian vein (high SVC). Each of the electrodes 40, 42, 44A-44D, 48, 50, 62, 66 and 67 may be electrically coupled to a respective one of the conductors within the lead body of its associated lead 18, 20, 22, and thereby individually coupled to the signal generator and sensing module of IMD 16.

In some examples, as illustrated in FIG. 2, IMD 16 includes one or more housing electrodes, such as housing electrode 58, which may be formed integrally with an outer surface of hermetically-sealed housing 60 of IMD 16 or otherwise coupled to housing 60. In some examples, housing electrode 58 is defined by an uninsulated portion of an outward facing portion of housing 60 of IMD 16. Other division between insulated and uninsulated portions of housing 60 may be employed to define two or more housing electrodes. In some examples, housing electrode 58 comprises substantially all of housing 60.

IMD 16 may sense electrical signals attendant to the depolarization and repolarization of heart 12 via electrodes 40, 42, 44A-44D, 48, 50, 58, 62, 66 and 67. The electrical signals are conducted to IMD 16 via the respective leads 18, 20, 22, or in the case of housing electrode 58, a conductor coupled to the housing electrode. IMD 16 may sense such electrical signals via any bipolar combination of electrodes 40, 42, 44A-44D, 48, 50, 58, 62, 66 and 67. Furthermore, any of the electrodes 40, 42, 44A-44D, 48, 50, 58, 62, 66 and 67 may be used for unipolar sensing in combination with housing electrode 58.

In some examples, IMD 16 delivers pacing pulses via bipolar combinations of electrodes 40, 42, 44A-44D, 48 and 50 to produce depolarization of cardiac tissue of heart 12. In some examples, IMD 16 delivers pacing pulses via any of electrodes 40, 42, 44A-44D, 48, 50, and 62 in combination with housing electrode 58 in a unipolar configuration. For example, electrodes 40, 42, and/or 58 or 62 or 40 may be used to deliver RV pacing to heart 12. Additionally or alternatively, electrodes 44A-44D and/or 58 may be used to deliver LV pacing to heart 12, and electrodes 48, 50 and/or 58 may be used to deliver RA pacing to heart 12.

Furthermore, IMD 16 may deliver defibrillation pulses to heart 12 via any combination of elongated electrodes 62, 66 and 67, and housing electrode 58. Electrodes 58, 62, and 66 and 67 may also be used to deliver cardioversion pulses to heart 12. Electrodes 62, 66 and 67 may be fabricated from any suitable electrically conductive material, such as, but not limited to, platinum, platinum alloy or other materials known to be usable in implantable defibrillation electrodes.

The configuration of therapy system 10 illustrated in FIGS. 1 and 2 is merely one example. In other examples, a therapy system may include epicardial leads and/or patch electrodes instead of or in addition to the transvenous leads 18, 20, 22 illustrated in FIGS. 1 and 2. Further, IMD 16 need not be implanted within patient 14. In examples in which IMD 16 is not implanted in patient 14, IMD 16 may deliver defibrillation pulses and other therapies to heart 12 via percutaneous leads that extend through the skin of patient 14 to a variety of positions within or outside of heart 12.

In addition, in other examples, a therapy system may include any suitable number of leads coupled to IMD 16, and each of the leads may extend to any location within or proximate to heart 12. For example, other examples of therapy systems may include three transvenous leads located as illustrated in FIGS. 1 and 2, and an additional lead located within or proximate to left atrium 36.

Two or more electrodes, and the polarity of the electrodes, define a vector, or path, for delivering pacing pulses to heart 12. As described above, there are numerous vectors that may be used to deliver pacing pulses to heart 12. For example, various combinations of the electrodes on a single quadripolar lead, i.e., a lead with four electrodes on the lead, such as lead 20, as well as combinations of the lead electrodes with an electrode on the housing of an IMD 16 may provide sixteen or more different vectors that may be used to deliver pacing pulses to a chamber of heart 12 that the lead is within or on. Testing each vector in order to determine which vector at a particular voltage amplitude provides synchrony between chambers of the heart, does not cause phrenic nerve stimulation, and sufficiently captures the heart without unnecessarily depleting the battery, e.g., by pacing at too high a voltage, may be a time-consuming process.

Using the techniques of this disclosure, a user, e.g., a clinician, may quickly determine one or more electrode combinations of one or more leads of an implantable medical device that have acceptable user selected criteria, as automatically assessed and prioritized by a processor. As described in more detail below, various techniques of this disclosure utilize a combination of measurements of the user specified criteria to determine the one or more electrode combinations by which to pace the heart. For example, the techniques may include measuring or assessing the user selected criteria such as CRT efficacy, capture threshold amplitudes, R-wave amplitudes, phrenic nerve stimulation amplitudes, longevity, and impedances, and then automatically prioritizing the tested vectors based on the measurements.

In some example implementations, the techniques may prioritize the test vectors based on a previously defined or specified priority order. For example, a user may specify that the vectors should be prioritized using the measured values of the selected criteria in the following order: CRT efficacy, capture threshold amplitude, R-wave amplitude, and impedance.

In other example implementations, the techniques may include associating a range of values with one or more of the criteria such that a measured value within the range of values reduces the desirability of the tested vector and thus lowers the priority of the vector. For instance, a vector may not be desirable even though the vector had a low capture threshold if the measured impedance of that vector fell outside of a range of values of acceptable impedance values, as described in more detail below.

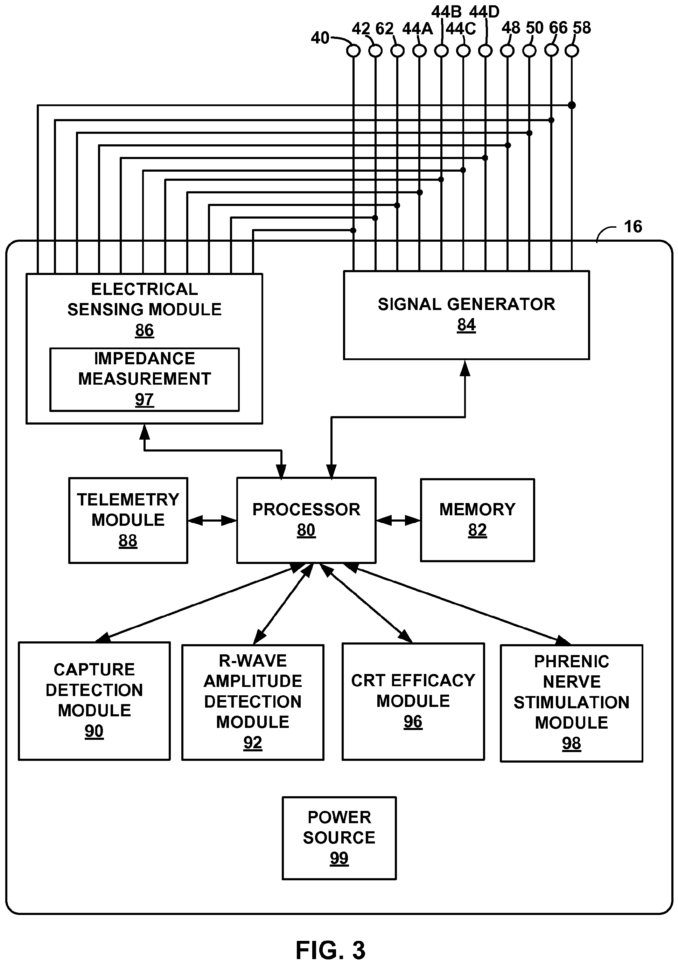

FIG. 3 is a block diagram illustrating one example configuration of IMD 16. In the example illustrated by FIG. 3, IMD 16 includes a processor 80, memory 82, signal generator 84, electrical sensing module 86, and telemetry module 88. IMD 16 further includes capture detection module 90, R-wave amplitude detection module 92, CRT efficacy module 96, and phrenic nerve stimulation module 98, in order to assess, or measure, criteria such as capture thresholds, R-wave amplitudes, CRT efficacy, phrenic nerve stimulation, impedance, and longevity. Using various techniques of this disclosure, a processor, e.g., processor 80, may prioritize tested vectors based on the measured criteria.

Memory 82 may include computer-readable instructions that, when executed by processor 80, cause IMD 16 and processor 80 to perform various functions attributed throughout this disclosure to IMD 16, processor 80, capture detection module 90, R-wave amplitude detection module 92, CRT efficacy evaluation module 96, and phrenic nerve stimulation module 98. The computer-readable instructions may be encoded within memory 82. Memory 82 may comprise computer-readable storage media including any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

Power source 99 may be a non-rechargeable primary cell battery or a rechargeable battery and may be coupled to power circuitry. However, the disclosure is not limited to examples in which the power source is a battery. In another example, power source 99 may comprise a supercapacitor. In some examples, power source 99 may be rechargeable via induction or ultrasonic energy transmission, and include an appropriate circuit for recovering transcutaneously received energy. For example, power source 99 may be coupled to a secondary coil and a rectifier circuit for inductive energy transfer. In additional examples, power source 99 may include a small rechargeable circuit and a power generation circuit to produce the operating power.

Processor 80 may include any one or more of a microprocessor, a controller, a digital signal processor (DSP), an application specific integrated circuit (ASIC), a field-programmable gate array (FPGA), or equivalent discrete or integrated logic circuitry. In some examples, processor 80 may include multiple components, such as any combination of one or more microprocessors, one or more controllers, one or more DSPs, one or more ASICs, or one or more FPGAs, as well as other discrete or integrated logic circuitry. The functions attributed to processor 80 herein may be embodied as software, firmware, hardware or any combination thereof. In one example, capture detection module 90, R-wave amplitude detection module 92, and CRT efficacy evaluation module 96, impedance measurement module 97, and phrenic nerve stimulation module 98 may be stored or encoded as instructions in memory 82 that are executed by processor 80.

Processor 80 controls signal generator 84 to deliver stimulation therapy, e.g., cardiac pacing or CRT, to heart 12 according to a selected one or more therapy programs, which may be stored in memory 82. Signal generator 84 is electrically coupled to electrodes 40, 42, 44A-44D, 48, 50, 58, 62, and 66, e.g., via conductors of the respective lead 18, 20, 22, or, in the case of housing electrode 58, via an electrical conductor disposed within housing 60 of IMD 16. Signal generator 84 is configured to generate and deliver electrical stimulation therapy to heart 12 via selected combinations of electrodes 40, 42, 44A-44D, 48, 50, 58, 62, 66, and 67. In some examples, signal generator 84 is configured to deliver cardiac pacing pulses. In other examples, signal generator 84 may deliver pacing or other types of stimulation in the form of other signals, such as sine waves, square waves, or other substantially continuous time signals.

Stimulation generator 84 may include a switch module (not shown) and processor 80 may use the switch module to select, e.g., via a data/address bus, which of the available electrodes are used to deliver pacing pulses. Processor 80 may also control which of electrodes 40, 42, 44A-44D, 48, 50, 58, 62, 66, and 67 is coupled to signal generator 84 for generating stimulus pulses, e.g., via the switch module. The switch module may include a switch array, switch matrix, multiplexer, or any other type of switching device suitable to selectively couple a signal to selected electrodes.

Electrical sensing module 86 monitors signals from at least one of electrodes 40, 42, 44A-44D, 48, 50, 58, 62, 66, or 67 in order to monitor electrical activity of heart 12. Electrical sensing module 86 may also include a switch module to select which of the available electrodes are used to sense the cardiac activity. In some examples, processor 80 selects the electrodes that function as sense electrodes, or the sensing vector, via the switch module within electrical sensing module 86.

Electrical sensing module 86 includes multiple detection channels, each of which may be selectively coupled to respective combinations of electrodes 40, 42, 44A-44D, 48, 50, 58, 62, 66, or 67 to detect electrical activity of a particular chamber of heart 12. Each detection channel may comprise an amplifier that outputs an indication to processor 80 in response to detection of an event, such as a depolarization, in the respective chamber of heart 12. In this manner, processor 80 may detect the occurrence of R-waves and P-waves in the various chambers of heart 12.

Memory 82 stores intervals, counters, or other data used by processor 80 to control the delivery of pacing pulses by signal generator 84. Such data may include intervals and counters used by processor 80 to control the delivery pacing pulses to one or both of the left and right ventricles for CRT. The intervals and/or counters are, in some examples, used by processor 80 to control the timing of delivery of pacing pulses relative to an intrinsic or paced event, e.g., in another chamber.

Capture detection module 90, in the example of FIG. 3, is capable of detecting capture threshold amplitudes and loss-of-capture (LOC) during capture detection tests. Any of a variety of techniques may be used to detect capture, such as detecting evoked responses, detecting mechanical contraction responsive to pacing stimuli, and/or detecting activation in another chamber responsive to delivery of pacing stimuli in a first chamber.

In one example, capture detection module 90 uses signals from electrical sensing module 86 to detect capture and/or inadequate capture when signal generator 84 delivers a pacing pulse. Via the switching module, processor 80 may control which of electrodes 40, 42, 44A-44D, 48, 50, 58, 62, 66, and 67 is coupled to electrical sensing module 86 to detect a response subsequent to the delivery of a pacing pulse for the determination of whether the pacing pulse captured the heart. Memory 82 may store predetermined intervals or voltage thresholds which define whether a detected signal has an adequate magnitude and is appropriately timed relative to the pacing pulse to be considered indicative of capture, e.g., an evoked response. In some examples, a channel of electrical sensing module 86 used to detect capture comprises an amplifier which provides an indication to processor 80 when a detected signal has an adequate magnitude.

Processor 80 controls the selection of electrode configurations for delivering pacing pulses and for detecting capture and/or LOC. Processor 80, for example, may communicate with signal generator 84 to select two or more stimulation electrodes in order to generate one or more pacing pulses for delivery to a selected chamber of heart 12. Processor 80 may also communicate with electrical sensing module 86 to select two or more sensing electrodes for capture detection based on the chamber to which the pacing pulse is delivered by signal generator 84.

Processor 80 controls electrical sensing module 86 and R-wave amplitude detection module 92 to sense and detect the amplitude of any R-waves. In particular, R-wave amplitude detection module 92 detects the amplitude of an R-wave caused by the delivered pacing stimulus via the vector. The higher the R-wave amplitude, the better the IMD's ability to sense.

IMD 16 further includes CRT efficacy evaluation module 96 for measuring criteria indicative of the efficiency of contraction of heart 12 of patient 14, and determining atrioventricular or interventricular intervals for delivery of pacing pulses to achieve synchrony and efficient contraction, thereby maximizing the output of heart 12. CRT efficacy evaluation module 96 may implement various CRT efficacy techniques, such as a time-based algorithm or fusion pacing. Module 96 may utilize a variety of different criteria to evaluate the efficacy of CRT, including ejection times, a time derivative of intercardiac pressure (dP/dt), echo-cardiogram, or the relative timing of electrical depolarizations of the heart.

As indicated above, impedance is another criterion by which a vector may be evaluated and prioritized. IMD 16 and, in particular, sensing module 86 further includes impedance measurement module 97 for measuring the impedance of one or more vectors. In some examples, sensing module 86 and/or processor 80 are capable of measuring impedance for any of a variety of electrical paths that include two or more of electrodes, e.g., at least one of electrodes 44A-44D in combination with one of electrodes 40, 42, 48, 50, 58, 62, 66, and 67. In the illustrated example of FIG. 3, sensing module 86 comprises impedance measurement module 97, which may measure electrical parameter values during delivery of an electrical signal between at least two of the electrodes. Processor 80 may control signal generator 84 to deliver the electrical signal between the electrodes. Processor 80 may determine impedance values based on parameter values measured by impedance measurement module 97, and store measured impedance values in memory 82.

In some examples, processor 80 may perform an impedance measurement by controlling delivery, from signal generator 84, of a voltage pulse between first and second electrodes. Measurement module 97 may measure a resulting current, and processor 80 may calculate an impedance value that includes both a resistive and a reactive component based upon the voltage amplitude of the pulse and the measured amplitude of the resulting current. In other examples, processor 80 may perform an impedance measurement by controlling delivery, from signal generator 84, of a current pulse between first and second electrodes. Measurement module 97 may measure a resulting voltage, and processor 80 may calculate an impedance value based upon the current amplitude of the pulse and the measured amplitude of the resulting voltage. Measurement module 97 may include circuitry for measuring amplitudes of resulting currents or voltages, such as sample and hold circuitry.

In these examples, signal generator 84 delivers signals that do not necessarily deliver stimulation therapy to heart 12, due to, for example, the amplitudes of such signals and/or the timing of delivery of such signals. For example, these signals may comprise sub-threshold amplitude signals that may not stimulate heart 12. In some cases, these signals may be delivered during a refractory period, in which case they also may not stimulate heart 12. IMD 16 may use defined or predetermined pulse amplitudes, widths, frequencies, or electrode polarities for the pulses delivered for these various impedance measurements. In some examples, the amplitudes and/or widths of the pulses may be sub-threshold, e.g., below a threshold necessary to capture or otherwise activate tissue, such as cardiac tissue of heart 12.

In certain cases, IMD 16 may collect impedance values that include both a resistive and a reactive (i.e., phase) component. In such cases, IMD 16 may measure impedance during delivery of a sinusoidal or other time varying signal by signal generator 84, for example. Thus, as used in this disclosure, the term "impedance" is used in a broad sense to indicate any collected, measured, and/or calculated value that may include one or both of resistive and reactive components.

As indicated above, power source longevity, or simply longevity, is another criterion by which a vector may be evaluated and prioritized. Longevity is a function of the pacing output and impedance. Vectors delivered having higher pacing amplitudes and lower impedances result in more current drain and negatively affect the longevity of power source 99. As such, these vectors deplete power source 99 more quickly than vectors with lower pacing amplitudes and higher impedance. Using various techniques of this disclosure, a processor may determine a "longevity" criterion for a vector based on an amplitude of the pacing stimulus and an impedance. Vectors that have a longevity criterion which would extend the life of power source 99 may be prioritized above vectors having a longevity criterion which would deplete the life of power source 99.

Using various techniques of this disclosure, capture detection module 90, R-wave amplitude detection module 92, CRT efficacy evaluation module 96, and impedance measurement module 98 may determine criteria such as capture detection thresholds, R-wave amplitudes, CRT efficacy, impedance values, and longevity by which processor 80 may prioritize a plurality of tested vectors, as described in more detail below. In addition, in some example implementations, phrenic nerve stimulation may be used as another criterion by which processor 80 may prioritize a plurality of tested vectors. Phrenic nerve stimulation is generally undesirable during pacing therapy. For example, phrenic nerve stimulation may cause a hiccup each time a stimulation signal is delivered to stimulate LV contraction, e.g., with each heart beat. It may be desirable to selectively stimulate the myocardium of the LV of heart 12 without stimulating the phrenic nerve. Accordingly, a plurality of vectors may be evaluated to assess their effects on a patient's phrenic nerve.

For example, to test a vector, processor 80 may communicate with signal generator 84 to select two stimulation electrodes in order to generate one or more pacing pulses for delivery to a selected chamber of heart 12. Phrenic nerve stimulation module 98 may assess phrenic nerve stimulation measurements in order to determine if the vector stimulated the phrenic nerve. For example, phrenic nerve stimulation module 98 may automatically determine if a vector stimulated the phrenic nerve using various techniques such as assessment of the following: heart sounds, accelerometer data, transthoracic impedance measurements, and electrograms, e.g., by detection of rhythmic, motion-based noise in a cardiac electrogram. In addition, phrenic nerve stimulation may be determined manually by clinician or patient verification, e.g., if a clinician or patient notices the patient hiccup upon delivery of a pacing pulse.

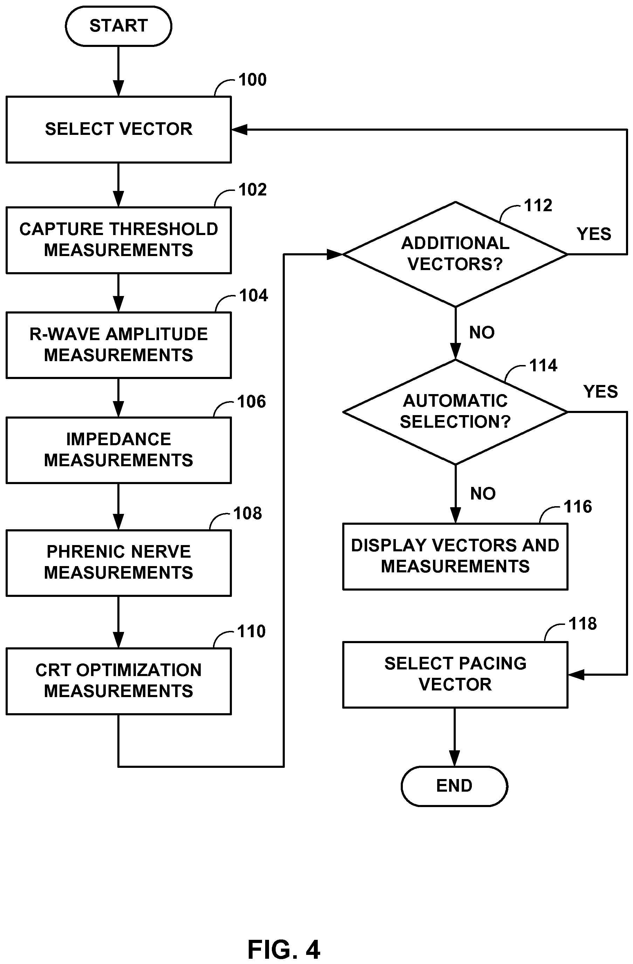

FIG. 4 is a flow diagram illustrating an example method for assessing a plurality of vectors in accordance with this description. In FIG. 4, pacing is turned on and one of a plurality of vectors is selected for testing (100) in, for example, an LV bipolar configuration or an LV to RV coil configuration or unipolar. For example, in a bipolar configuration, electrode 44A may be selected as a cathode to sink current that is sourced from one of electrodes 44B-44D configured as an anode. In an LV to RV coil configuration, electrode 44A may be selected as a cathode to sink current that is sourced from an anode on RV lead 18, such as electrode 62 or unipolar to 58. The vector may be selected for testing either automatically by processor 80 (FIG. 3) or manually by a user.

Following selection of a vector, various criteria are assessed by which the vector may be prioritized, e.g., automatically by processor 80 or manually by a user. In FIG. 4, processor 80 controls signal generator 84 to deliver a pacing stimulus to heart 12. Electrical sensing module 86 monitors electrical activity in heart 12 and, based on the monitored activity, capture detection module 90 determines a capture threshold (102). For example, capture detection module 90 detects whether the pacing stimulus has captured the heart and, if so, the magnitude of the pacing stimulus, e.g., a voltage magnitude or pulse width. In some example implementations, for each vector, processor 80 controls signal generator 84 to iteratively increase and/or decrease the magnitude of pacing pulses until capture detection module 90 determines that capture is achieved or lost to identify the capture threshold for the vector.

In some example implementations, the capture threshold determination is automatic. In other example implementations, the capture threshold determination is manual, i.e., performed by a clinician. In some examples, in accordance with this disclosure, a user may select one or more of the criteria by which each of the plurality of vectors may be prioritized.

Processor 80 then controls R-wave amplitude detection module 92 to detect the amplitude of any R-waves (104) in order to determine whether an electrode is positioned over viable tissue, e.g., an electrode is not positioned over ischemic tissue.

Still referring to FIG. 4, processor 80 may determine an impedance value of the vector based on parameter values measured by impedance measurement module 97 (106). Impedance measurements may be desirable in that these measurements may help in determining whether the impedance of the vector is within the programmable range of the IMD 16. Impedance measurements are also a factor in power source longevity, which, as described above, may be used as another criterion (not depicted).

Next, phrenic nerve stimulation module 98 may assess phrenic nerve stimulation measurements in order to determine if the vector stimulated the phrenic nerve (108). For example, phrenic nerve stimulation module 98 may automatically determine if a vector stimulated the phrenic nerve using various techniques such as assessment of the following: ejection times, transthoracic impedance measurements, heart sounds, accelerometer data, patient input, and electrograms. In addition, phrenic nerve stimulation may be determined manually by clinician verification, e.g., if a clinician sees the patient hiccup upon delivery of a pacing pulse.

Another criterion used for assessment of a vector is CRT efficacy measurements (110). CRT efficacy evaluation module 96 measures criteria indicative of the efficiency of contraction of heart 12, and determines atrioventricular or interventricular intervals for delivery of pacing pulses to achieve synchrony and efficient contract, thereby maximizing the output of heart 12. CRT efficacy evaluation module 96 may implement various CRT efficacy evaluation techniques such as a time-based algorithm or fusion pacing. Module 96 may utilize a variety of different criteria to evaluate the efficacy of CRT, including ejection times, a time derivative of intercardiac pressure (dP/dt), echo-cardiogram, or the relative timing of electrical depolarizations of the heart. Thus, for each of the plurality of vectors, IMD 16 delivers a pacing stimulus and measures one or more selected criteria.

It should be noted that the criteria measurements depicted in FIG. 4 at 102-110 need not be performed in the order shown in FIG. 4 or as described above. In addition, all of the criteria measurements depicted in FIG. 4 at 102-110 need not be performed. Rather, in accordance with this disclosure, a user, e.g., a clinician, may select particular criteria to be measured, e.g., only capture thresholds and phrenic nerve thresholds, as will be described in more detail below.

Once the particular criteria have been measured, processor 80 stores the measurements of the criteria for that particular vector in memory 82. If there are additional vectors to be tested ("YES" branch of block 112), then another vector is selected at 100 (either automatically by processor 80 or manually by the user). Upon selection of another vector, each of the selected criteria is measured, as described above with respect to steps 102-110.

Vector selection at block 114 may be either automatic or manual. Manual vector selection ("NO" branch at block 114) allows the user, e.g., clinician, to manually select a vector configuration using the displayed vectors and measurements (116). In some example implementations, vectors and measurements may be communicated to a user via other means, such as via audio or printing. Automatic vector selection ("YES" branch at block 114) results in processor 80 automatically selecting the most desirable vector based on the determined prioritization (118). Thus, processor 80 automatically prioritizes the plurality of vectors based on the measurement of the one or more selected criteria.

If there are no additional vectors to be tested ("NO" branch of block 112) and if vector selection is set for automatic selection ("YES" branch of block 114), then processor 80 automatically selects one of the plurality of tested vectors for pacing based on either a default prioritization or a user-specified prioritization of the criteria. For example, one prioritization may prioritize or rank criteria in the following order: 1) CRT efficacy, 2) capture threshold amplitude, 3) R-wave amplitudes, and 4) impedance. In another example, criteria may be prioritized in the following order: 1) capture threshold amplitude, 2) phrenic nerve thresholds, 3) R-wave amplitudes, and 4) impedance. Of course, these are only two example prioritizations. In other examples, phrenic nerve stimulation amplitudes and/or longevity may also be included as selected criteria and prioritized by the user. Numerous other prioritizations are possible that may be user-selected, which will not be described.

If there are no additional vectors to be tested ("NO" branch of block 112) and if vector selection is not set for automatic selection ("NO" branch of block 114), e.g., vector selection is set for manual selection of vectors, then processor 80 controls a display, e.g., of programmer 24, to display or present the tested vectors and the measurements of the criteria measured at one or more of steps 102-110. In some example implementations, vectors and measurements may be communicated to a user via other means, such as via audio or printing.

By way of specific example, assume that a user selected to test four vectors. In particular, the user selected to test the following vectors: 1) electrode 44A (cathode) on LV lead 20 to electrode 62 (anode) on RV lead 18, 2) electrode 44B (cathode) on LV lead 20 to electrode 62 (anode) on RV lead 18, 3) electrode 44C (cathode) on LV lead 20 to electrode 62 (anode) on RV lead 18, and 4) electrode 44D (cathode) on LV lead 20 to electrode 62 (anode) on RV lead 18. Further assume that the user selected the following criteria and prioritization (either by selecting a default group of one or more criteria or by actively selecting the particular criteria in a group) by which to assess these vectors: 1) CRT efficacy, 2) capture threshold amplitude, 3) R-wave amplitudes, and 4) impedance. Once all the vectors have been tested ("NO" branch of block 112), processor 80 may display or present to the user the tested vectors and the measurements of the criteria tested, as shown in Table 1 below:

**TABLE 1**

| ELECTRODE | CRT EFFICACY | CAPTURE THRESHOLD | R-WAVE AMPLITUDE | IMPEDANCE |
|---|---|---|---|---|
| 44A | 1 | 2.0 V | 10 mV | 650 ohms |
| 44B | 3 | 1.8 V | 12 mV | 700 ohms |
| 44C | 4 | 2.5 V | 16 mV | 750 ohms |
| 44D | 2 | 5.5 V | 8 mV | 680 ohms |

Table 1 has five columns indicating, in order from left to right, the electrode tested, a CRT efficacy score, a capture threshold measurement (in volts), an R-wave amplitude measurement (in millivolts), and an impedance (in ohms). The electrodes are prioritized according to the measured criteria. In Table 1, processor 80 determined that, based on prioritization of the tested criteria, i.e., CRT efficacy is the most important of the four criteria and impedance is the least important of the four criteria, electrode 44A has the highest prioritization, i.e., the most desirable electrode of the four tested to select for pacing, followed by electrodes 44B and 44C, and lastly electrode 44D, i.e., the least desirable electrode of the four tested to select for pacing. In some examples, the clinician may prioritize the criteria.

It should be noted that a table is only one example manner in which results may be displayed to a user. In other examples, processor 80 may control a single vector to be displayed as the "best pacing vector." Or, processor 80 may control the top three vectors to be displayed.

As indicated above, a range of values may be associated with one or more of the criteria such that a measured value within the range of values reduces the desirability of the tested vector and thus lowers the priority of the vector. Example ranges of values that may lower the priority of a vector include the following: capture threshold amplitudes greater than about 3 V, R-wave amplitudes less than about 1 mV, impedance values less than about 200 ohms and greater than about 3000 ohms, and phrenic nerve amplitudes less than about 8-10 V. These ranges of values may be referred to as exclusion criteria. These values may be attained by user input and stored in memory, e.g., memory 82. In other examples, these values may be based on patient data, e.g., individual patient's or a human model, or ranges specific to the IMD 16.

Referring again to Table 1, even though CRT efficacy evaluation module 96 determined that electrode 44D ranked higher than electrodes 44B and 44C for CRT efficacy, processor 80 determined that electrode 44D should have a lower prioritization than electrodes 44B and 44C because of its high capture threshold amplitude (5.5 V). In other words, the high capture threshold amplitude of electrode 44D reduces its desirability despite its CRT efficacy score. Similarly, if the exclusion criteria applied to the measured R-wave amplitudes and impedance values, then processor 80 may lower the prioritization of the associated vector.

The example method depicted in FIG. 4 may be implemented as either an automatic or manual test. If the test is automatic, then the various modules described above with respect to FIG. 3 may automatically perform the measurements associated with each module. If the test is manual, then a user, e.g., a clinician, may, for example, manually step through one or more of measurements 102-110 of FIG. 4 and manually select additional vectors to test.

In addition, the example method depicted in FIG. 4 may be either a static or dynamic test. For example, if implemented as a static test, then IMD 16 may perform the measurements on a patient over a single session. However, if implemented as a dynamic test, then IMD 16 may perform the measurements on a patient periodically over one or more days and adjust the pacing vectors as the measurements change.

Although the prioritization techniques were described above with respect to electrodes on an LV lead, in other example implementations, the prioritization techniques may be similarly applied to electrodes on leads in other chambers of the heart, e.g., atrial leads or RV leads.

Using the techniques depicted and described above with respect to FIG. 4, a clinician may quickly determine one or more electrode combinations of one or more leads of an implantable medical device for pacing. The method depicted in FIG. 4 may facilitate selection of a pacing vector by automating the measurements of one or more user-selectable prioritized criteria for each of a plurality of vectors.

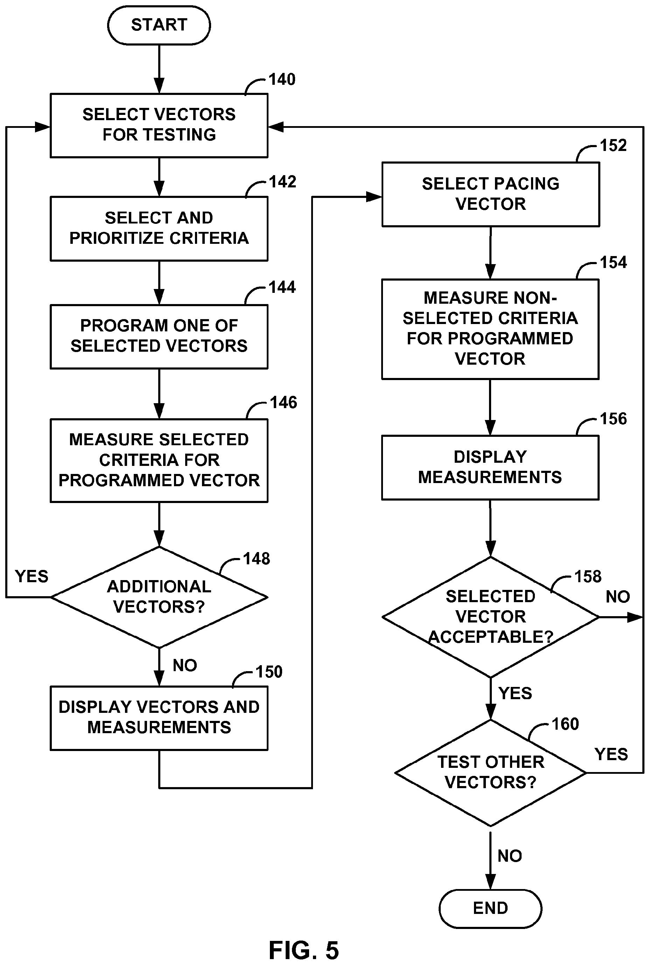

FIG. 5 is a flow diagram illustrating another example method for assessing a plurality of vectors in accordance with this disclosure. In some implementations, the method depicted in FIG. 5 may result in a faster selection of vectors than the method depicted in FIG. 4. For example, as described in more detail below, a user, e.g., a clinician, may select to test only a few available vectors and select to test only a few criteria for the selected vectors, thereby expediting the selection of vectors.

In FIG. 5, a user selects one or more vectors for testing (140). For example, the user may select to test all available vectors, only extended bipolar vectors (LV to RV coil), or unipolar adjacent electrode pairs, reverse polarities, particular vectors of interest, or only one vector. After selecting one or more vectors for testing, the user may select and prioritize one or more criteria by which processor 80 will assess the selected one or more vectors (142). For example, a user may have selected five vectors for testing at step 140, and at step 142 the user may select CRT efficacy as the sole criterion by which processor 80 will assess the five selected vectors. Or, the user may select an alternative criterion or one or more additional criteria such as capture threshold amplitude, phrenic nerve thresholds, R-wave amplitudes, and impedance. If two or more criteria are selected the user may prioritize the selected criteria. Selecting fewer criteria or fewer vectors may result in a faster test and thus expedite selection of a vector.

Once the user has selected the vectors (140) and selected and prioritized the criteria (142), pacing is turned on and processor 80 programs signal generator 84 to test one of the selected vectors (144). Depending on the criteria selected, one or more of capture detection module 90, R-wave amplitude detection module 92, CRT efficacy evaluation module 96, longevity, impedance measurement module 97, and phrenic nerve stimulation module 98 measure the selected criteria for the programmed vector (146).

If the user selected more than one vector to be tested ("YES" branch of block 148), processor 80 programs signal generator 84 to test another of the selected vectors and one or more of capture detection module 90, R-wave amplitude detection module 92, CRT efficacy evaluation module 96, impedance measurement module 97, and phrenic nerve stimulation module 98 measure the selected criteria for the programmed vector. If there are no additional vectors to be tested ("NO" branch of block 148), then processor 80 controls a display, e.g., of programmer 24, to display or present the tested vectors and the measurements of the criteria (150). The display may present the results in a table, processor 80 may control a single vector to be displayed as the "best pacing vector," processor 80 may control the top three vectors to be displayed, or the results may be presented to the user in some other manner. In this manner, processor 80 automatically prioritizes the vectors based on the measurements of the selected criteria. Following the display of the vectors and measurements, either processor 80 (automatically) or a user (manually) selects a pacing vector based on the measured criteria and user-specified prioritization of the selected criteria (152).

After either automatic or manual selection of a vector, one or more of capture detection module 90, R-wave amplitude detection module 92, CRT efficacy evaluation module 96, impedance measurement module 97, and phrenic nerve stimulation module 98 may measure the criteria that the user did not select at step 142 for the selected vector (154), and processor 80 may control a display to present the measurements of the criteria (156). If the user determines that the selected vector is not acceptable after reviewing the measurements of the criteria that the user did not initially select at step 142 ("NO" branch at block 158), then the user may select another vector for testing at 140, or go to the next highest prioritized vector out of the previous testing.

If the user determines, or confirms, that the selected vector is acceptable after reviewing the measurements of the criteria that the user did not initially select at step 142 ("YES" branch at block 158) and the user would like to test other vectors ("YES" branch at block 160), then the user may select another vector for testing at 140. If the user determines, or confirms, that the selected vector is acceptable after reviewing the measurements of the criteria that the user did not initially select at step 142 ("YES" branch at block 158) and the user does not want to test other vectors ("NO" branch at block 160), then processor 80 stores the selected vector in memory 82 and controls signal generator 84 to apply pacing therapy using the selected vector.

Again, the techniques described with respect to FIG. 5 may expedite selection of a pacing vector by allowing a user to test a portion of the available criteria and vectors.

The techniques presented above have been described with respect to facilitating selection of a pacing vector on a multi-electrode lead such as quadripolar lead 20 of FIG. 2. However, the techniques described above may also be applied to other multipolar leads, as shown and described in more detail below.

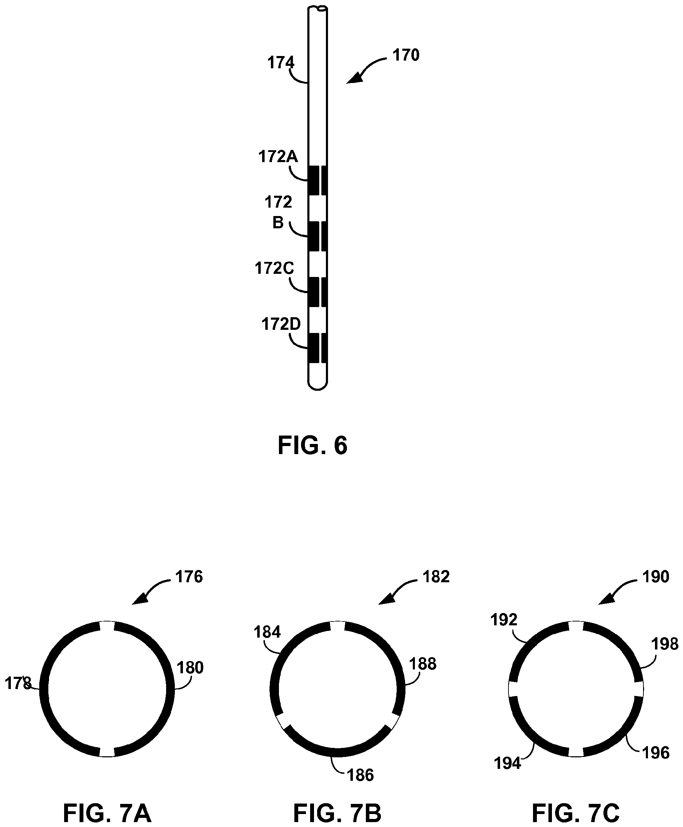

FIG. 6 is a conceptual diagram illustrating one example of an implantable multipolar stimulation lead. In particular, lead 170 is an example of a multipolar lead that includes four electrode levels, or bands, 172 (includes bands 172A-172D) mounted at various positions along the axial length of lead housing 174. Bands 172A, 172B, 172C, and 172D may be equally spaced along a distal portion of the axial length of lead housing 174, e.g., as illustrated in FIG. 6. Each of bands 172 may have two or more electrodes located at different angular positions around the circumference of lead housing 30, as shown and described below with respect to FIGS. 7A-7C.

FIGS. 7A-7C are transverse cross-sections of example multipolar stimulation leads having two or more electrodes around the circumference of the lead. FIG. 7A shows band 176 which includes two electrodes 178 and 180. Each electrode 178 and 180 wraps approximately 170 degrees around the circumference of band 176. Spaces of approximately 10 degrees are located between electrodes 178 and 180 to prevent inadvertent coupling of electrical current between the electrodes. Each electrode 178 and 180 may be programmed to act as an anode or cathode.

FIG. 7B shows band 182 which includes three equally sized electrodes 184, 186, and 188. Each electrode 184, 186 and 188 encompass approximately 110 degrees of the circumference of band 182. Similar to band 176, spaces of approximately 10 degrees separate electrodes 184, 186 and 188. Electrodes 184, 186 and 188 may be independently programmed as an anode or cathode for stimulation, or electrodes 184, 186, and 188 may be combined together to make a larger electrode.

FIG. 7C shows band 190 which includes four electrodes 192, 194, 196, and 198. Each electrode 192-198 covers approximately 80 degrees of the circumference with approximately 10 degrees of insulation space between adjacent electrodes. In other embodiments, up to ten or more electrodes may be included within an electrode level. In alternative embodiments, consecutive bands of lead 170 of FIG. 6 may include a variety of bands 176, 182, and 190, and lead 170 may include more bands then are shown in FIG. 6. The above-described sizes of electrodes within a band are merely examples, and various techniques of this disclosure are not limited to the example electrode sizes.

Multipolar stimulation leads having two or more electrodes around the circumference of the lead, as in FIGS. 7A-7C, for example, may be useful in minimizing or eliminating phrenic nerve stimulation. For example, if processor 80 configures all the electrodes of a band to act as cathodes, e.g., electrodes 192-196 of band 190 in FIG. 7C, and phrenic nerve stimulation module 98 detects that a pacing stimulus delivered by all the electrodes of the band results in phrenic nerve stimulation, then processor 80 may individually test each of electrodes of the band to determine if any of these electrodes or combinations reduce or eliminate phrenic nerve stimulation. Delivering a pacing stimulus via an electrode within a band, e.g., electrode 192 of band 190 of FIG. 7C, may be referred to as "intraband" pacing.

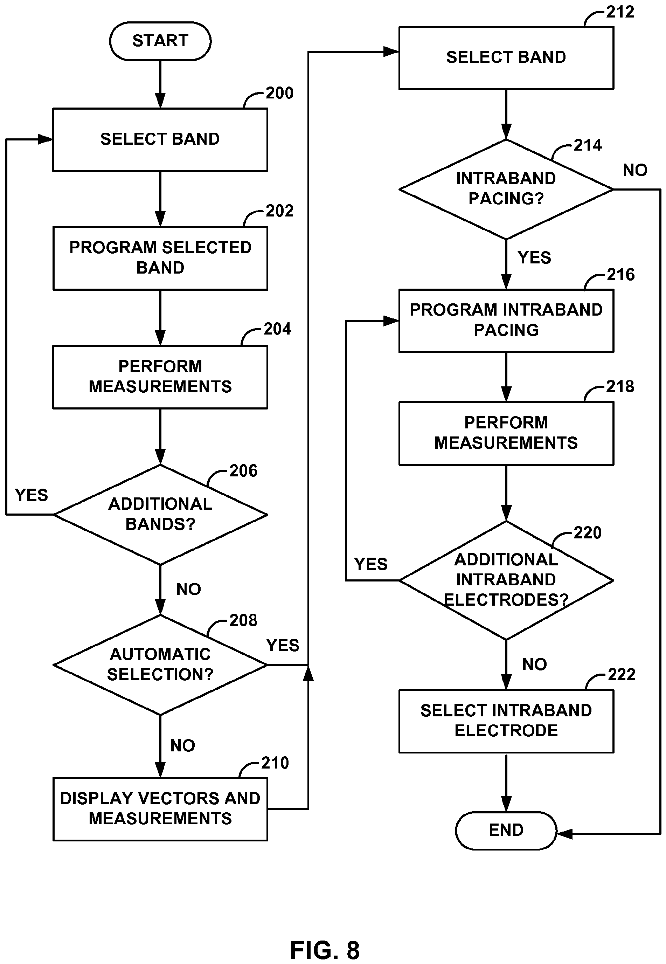

In accordance with certain techniques of this disclosure, a processor, e.g., processor 80, may first prioritize one of a plurality of bands, e.g., band 172B of FIG. 6, for delivering pacing stimuli, and then prioritize one of a plurality of electrodes, e.g., electrode 192, within the prioritized band. In this manner, the processor may determine the most effective electrode of the most effective band by which pacing stimuli should be delivered to a heart, as described below with respect to FIG. 8.

FIG. 8 is a flow diagram illustrating another example method for assessing a plurality of vectors in accordance with this disclosure. In particular, FIG. 8 depicts an example method for use with multipolar stimulation leads, e.g., stimulation leads having multiple bands and having two or more electrodes around the circumference of the lead and forming a band. For example, if pacing stimuli delivered via a particular band of the multipolar lead cause phrenic nerve stimulation, then the example method shown in FIG. 8 may be used to determine which electrode in the band does not cause phrenic nerve stimulation, thereby allowing the use of "intraband" pacing for delivery of pacing stimuli to a heart.

In FIG. 8, pacing is turned on and one of a plurality of bands on the multipolar lead is selected for testing (200) in either an LV bipolar configuration or an LV to RV coil or unipolar configuration. For example, in a bipolar configuration, each of electrodes 192-198 of band 190 in FIG. 7C may be configured as a cathode to sink current that is sourced from another band on the lead that is configured as an anode. In an LV to RV coil configuration, each of electrodes 192-198 of band 190 in FIG. 7C may be selected as a cathode to sink current that is sourced from an anode on RV lead 18. The band may be selected for testing either automatically by processor 80 (FIG. 3) or manually by a user.

Following selection of a band, processor 80 controls signal generator 84 to program the selected band and various criteria are measured and assessed by which the band may be prioritized, e.g., automatically by processor 80 or manually by a user. In FIG. 8, processor 80 controls signal generator 84 to deliver a pacing stimulus to heart 12. Electrical sensing module 86 monitors electrical activity in heart 12 and, based on the monitored activity, one or more of capture detection module 90, R-wave amplitude detection module 92, impedance measurement module 97, phrenic nerve stimulation module 98, and CRT efficacy evaluation module 96 perform measurements (204). The details of these modules and measurements were described above with respect to FIG. 4 and, for conciseness, will not be described again.

Once the particular criteria have been measured, processor 80 stores the measurements of the criteria for that particular vector in memory 82. If there are additional bands to be tested ("YES" branch of block 206), then another band is selected at 200 (either automatically by processor 80 or manually by the user). Upon selection of another band, each of the selected criteria is measured as described above.

If there are no additional bands to be tested ("NO" branch of block 206) and if band selection is set for automatic selection ("YES" branch of block 208), then processor 80 automatically selects one of the plurality of tested bands for pacing based on either a default prioritization or a user-specified prioritization of the criteria. For example, one prioritization may prioritize or rank criteria in the following order: 1) CRT efficacy, 2) capture threshold amplitude, 3) R-wave amplitudes, and 4) impedance. In another example, criteria may be prioritized in the following order: 1) capture threshold amplitude, 2) phrenic nerve thresholds, 3) R-wave amplitudes, and 4) impedance. Of course, these are only two example prioritizations. Numerous other prioritizations are possible that may be user-selected, which will not be described.

If there are no additional bands to be tested ("NO" branch of block 206) and if band selection is not set for automatic selection ("NO" branch of block 208), e.g., band selection is set for manual selection of bands, then processor 80 controls a display, e.g., of programmer 24, to display or present the tested bands and the measurements of the criteria measured at step 204. The display may present the results in a table, processor 80 may control a single vector to be displayed as the "best pacing vector," processor 80 may control the top three vectors to be displayed, or the results may be presented to the user in some other manner.

As indicated above, a range of values may be associated with one or more of the criteria such that a measured value within the range of values reduces the desirability of the tested vector and thus lowers the priority of the vector. Example ranges of values that may result in the processor lowering the priority of a vector include the following: capture threshold amplitudes greater than about 3 V, R-wave amplitudes less than about 1 mV, impedance values less than about 200 ohms and greater than about 3000 ohms, and phrenic nerve amplitudes less than about 10 V. These ranges of values may be referred to as exclusion criteria. In some examples, these ranges of values may be attained by user input and stored in memory, e.g., memory 82. In other examples, these values may be based on patient data, e.g., individual patient's or a human model, or ranges specific to the IMD 16.

It should be noted that in some examples, one or more of the criteria used for prioritization may be weighted. That is, a first criterion may be less favorable than a second criterion. As such, the first criterion may be weighted more heavily than the second criterion.

Once the processor, e.g., processor 80, has prioritized the bands, the processor determines whether intraband pacing may be desirable, based on the phrenic nerve thresholds measured by phrenic nerve stimulation module 98. The processor may determine that intraband pacing is not needed if phrenic nerve amplitudes are greater than about 10 V and if capture thresholds, impedance values, and R-wave amplitudes are within the above-mentioned exclusion criteria. If the processor determines that intraband pacing is not needed ("NO" branch at block 214), then the algorithm is finished and the processor determines that the band selected at block 212 should be used for delivering pacing stimuli to the heart.

If the processor determines that intraband pacing is needed ("YES" branch at block 214), then processor 80 controls signal generator 84 to program a vector for testing using intraband electrodes, e.g., electrodes 192-198 of band 190 of FIG. 7C. Using band 190 of FIG. 7C as one example, processor 80 may test various combinations of electrodes 192-198 as vectors. For example, processor 80 may configure electrode 192 as a cathode and then configure an anode using the following electrode combinations or individual electrodes: (1) 194, 196, 198; (2) 194, 196; (3) 196, 198; (4) 194, 198; (5) 194; (6) 196; and (7) 198. In other words, if processor 80 configures electrode 192 of band 190 of FIG. 7C as a cathode, processor 80 may test seven vectors. Similarly, if processor 80 configures electrode 194 as a cathode, processor 80 may test seven different vectors. In addition, fourteen more vectors are created if processor 80 configures electrode 196 and 198, individually, as cathodes. In some examples, a user may specify subsets of the available vectors for testing. For example, rather than testing all available 1:1, 1:2, and 1:3 electrode combinations, as listed above, a user may select to test only 1:1 electrode combinations, only 1:2 electrode combinations, only 1:3 electrode combinations, combinations of 1:1, 1:2, and 1:3 electrode combinations, or some other permutation of electrode combinations.

Once processor 80 programs a vector for intraband pacing (216), processor 80 controls signal generator 84 to deliver a pacing stimulus to heart 12. Electrical sensing module 86 monitors electrical activity in heart 12 and, based on the monitored activity, one or more of capture detection module 90, R-wave amplitude detection module 92, impedance measurement module 97, phrenic nerve stimulation module 98, and CRT efficacy evaluation module 96 perform measurements (218). As mentioned above, processor 80 may determine a power source longevity criterion using pacing output and impedance measurements. If there are additional intraband electrodes for testing ("YES" branch of block 220), then processor 80 programs another vector for testing and one or more of capture detection module 90, R-wave amplitude detection module 92, impedance measurement module 97, phrenic nerve stimulation module 98, and CRT efficacy evaluation module 96 perform measurements (218).

If there are no more intraband electrodes to be tested ("NO" branch at block 220), processor 80 selects one or more intraband electrodes for pacing (222). In this manner, the techniques described above with respect to FIG. 8 may be used to select one band from a plurality of bands on a multipolar lead, and select one or more intraband electrodes for delivering pacing therapy to a heart.

FIG. 9 is functional block diagram illustrating an example configuration of programmer 24. As shown in FIG. 9, programmer 24 may include a processor 310, memory 312, user interface 314, telemetry module 316, and power source 318. Programmer 24 may be a dedicated hardware device with dedicated software for programming of IMD 16. Alternatively, programmer 24 may be an off-the-shelf computing device running an application that enables programmer 24 to program IMD 16.

A user may use programmer 24 to select therapy programs (e.g., sets of stimulation parameters), generate new therapy programs, modify therapy programs through individual or global adjustments or transmit the new programs to a medical device, such as IMD 16 (FIG. 1). The clinician may interact with programmer 24 via user interface 314, which may include display to present graphical user interface to a user, and a keypad or another mechanism for receiving input from a user. The user, e.g., a clinician, may define or select vectors to be tested and/or input vector impedance values via user interface 314.

User interface 314 may display the vectors to be tested as well as the results of the prioritization techniques described above to the clinician. As described above, user interface 314 may, in some examples, display each vector tested, and the criteria tested for that vector, e.g., CRT efficacy, capture thresholds, R-wave amplitudes, phrenic nerve stimulation amplitudes, and impedance, in some order that the clinician may select or adjust. The results of the tests may also be stored within memory 112. User interface 314 may comprise a display screen as well as speakers for outputting an audio signal to the user. In addition, programmer 24 may be configured to print measurements, vectors, and the like, or include an interface for connecting programmer 24 to an output device for printing measurements, vectors, and the like.

Processor 310 can take the form one or more microprocessors, DSPs, ASICs, FPGAs, programmable logic circuitry, or the like, and the functions attributed to processor 310 herein may be embodied as hardware, firmware, software or any combination thereof. Memory 312 may store instructions that cause processor 310 to provide the functionality ascribed to programmer 24 herein, and information used by processor 310 to provide the functionality ascribed to programmer 24 herein. Memory 312 may include any fixed or removable magnetic, optical, or electrical media, such as RAM, ROM, CD-ROM, hard or floppy magnetic disks, EEPROM, or the like. Memory 312 may also include a removable memory portion that may be used to provide memory updates or increases in memory capacities. A removable memory may also allow patient data to be easily transferred to another computing device, or to be removed before programmer 24 is used to program therapy for another patient.

Programmer 24 may communicate wirelessly with IMD 16, such as using RF communication or proximal inductive interaction. This wireless communication is possible through the use of telemetry module 316, which may be coupled to an internal antenna or an external antenna. An external antenna that is coupled to programmer 24 may correspond to the programming head that may be placed over heart 12, as described above with reference to FIG. 1. Telemetry module 316 may be similar to telemetry module 88 of IMD 16 (FIG. 3).

Telemetry module 316 may also be configured to communicate with another computing device via wireless communication techniques, or direct communication through a wired connection. Examples of local wireless communication techniques that may be employed to facilitate communication between programmer 24 and another computing device include RF communication according to the 802.11 or Bluetooth specification sets, infrared communication, e.g., according to the IrDA standard, or other standard or proprietary telemetry protocols. In this manner, other external devices may be capable of communicating with programmer 24 without needing to establish a secure wireless connection. An additional computing device in communication with programmer 24 may be a networked device such as a server capable of processing information retrieved from IMD 16.

In some examples, processor 310 of programmer 24 and/or one or more processors of one or more networked computers may perform all or a portion of the techniques described herein with respect to processor 80 and IMD 16. For example, processor 310 or another processor may receive voltages or currents measured by IMD 16 to calculate impedance measurements, or may receive impedance measurements from IMD 16. Processor 310 or another processor may prioritize vectors using any of the techniques described in this disclosure. Power source 318 delivers operating power to the components of programmer 24.

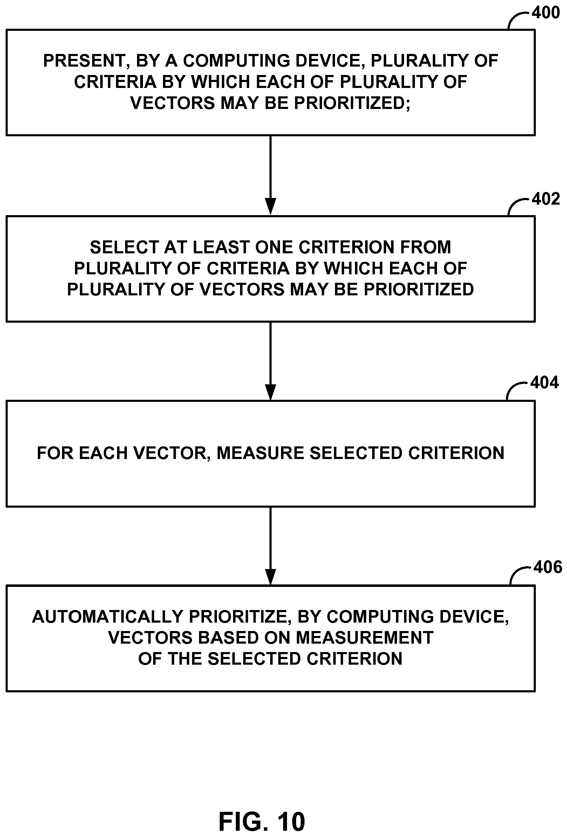

FIG. 10 is a flow diagram illustrating another example method for assessing a plurality of vectors in accordance with this disclosure. In particular, FIG. 10 depicts a method of facilitating selection of at least one vector from among a plurality of vectors for pacing a chamber of a heart. A computing device, e.g., programmer 24, presents a plurality of criteria to a user by which each of the plurality of vectors may be prioritized (400). A user selects at least one criterion from among a plurality of criteria by which each of the plurality of vectors may be prioritized (402). For example, a user may select one or more criteria such as, but not limited to, CRT efficacy, capture thresholds, R-wave amplitudes, phrenic nerve stimulation amplitudes, and impedance, by which the pacing vectors may be prioritized. Then, for each vector, a processor, e.g., processor 80, controls one or more of capture detection module 90, R-wave amplitude detection module 92, CRT efficacy module 96, phrenic nerve stimulation module 98, and impedance measurement module 97 to measure the one or more selected criteria (404). After performing the measurements for each vector, the processor of the computing device automatically prioritizes the plurality of vectors based on the measurement of the one or more selected criteria (406).

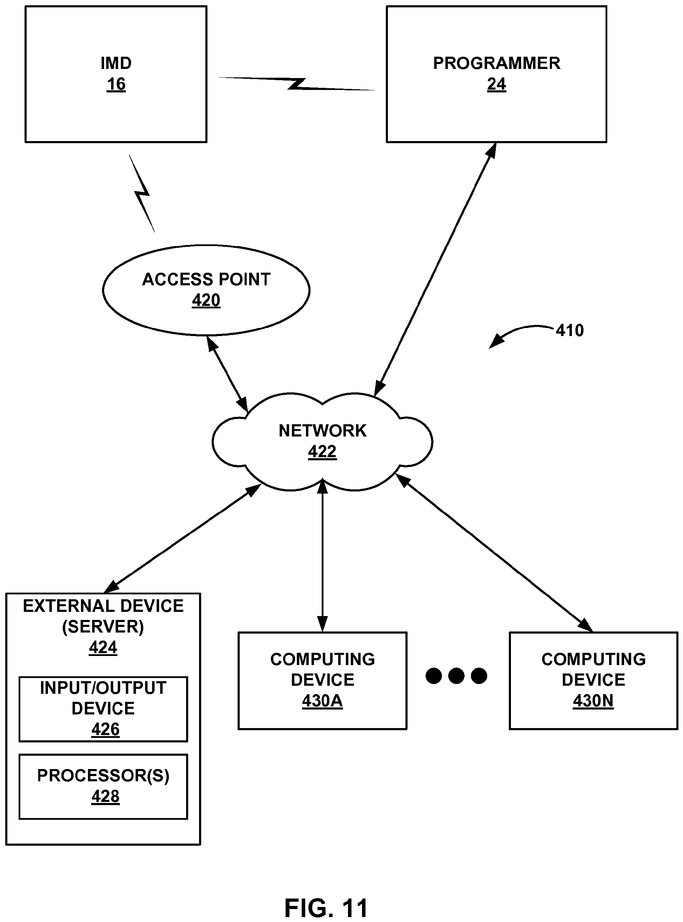

FIG. 11 is a block diagram illustrating an example system 410 that includes an external device, such as a server 424 having an input/output device 426 and processor(s) 428, and one or more computing devices 430A-430N, that are coupled to the IMD 16 and programmer 24 shown in FIG. 1 via a network 422. In this example, IMD 16 may use its telemetry module 88 to communicate with programmer 24 via a first wireless connection, and to communication with an access point 420 via a second wireless connection. In the example of FIG. 10, access point 420, programmer 24, server 424, and computing devices 430A-430N are interconnected, and able to communicate with each other, through network 422. In some cases, one or more of access point 420, programmer 24, server 424, and computing devices 430A-430N may be coupled to network 422 through one or more wireless connections. IMD 16, programmer 24, server 424, and computing devices 430A-430N may each comprise one or more processors, such as one or more microprocessors, DSPs, ASICs, FPGAs, programmable logic circuitry, or the like, that may perform various functions and operations, such as those described herein.

Access point 420 may comprise a device that connects to network 422 via any of a variety of connections, such as telephone dial-up, digital subscriber line (DSL), or cable modem connections. In other examples, access point 420 may be coupled to network 422 through different forms of connections, including wired or wireless connections. In some examples, access point 420 may be co-located with patient 14 and may comprise one or more programming units and/or computing devices (e.g., one or more monitoring units) that may perform various functions and operations described herein. For example, access point 420 may include a home-monitoring unit that is co-located with patient 14 and that may monitor the activity of IMD 16.

In some cases, server 424 may be configured to provide a secure storage site for data that has been collected from IMD 16 and/or programmer 24. Network 422 may comprise a local area network, wide area network, or global network, such as the Internet. In some cases, programmer 24 or server 424 may assemble data in web pages or other documents for viewing by trained professionals, such as clinicians, via viewing terminals associated with computing devices 430A-430N. The illustrated system of FIG. 10 may be implemented, in some aspects, with general network technology and functionality similar to that provided by the Medtronic CareLink® Network developed by Medtronic, Inc., of Minneapolis, MN.

In some examples, processor(s) 428 of server 424 may be configured to receive measurements of one or more of capture detection module 90, R-wave amplitude detection module 92, CRT efficacy evaluation module 96, impedance measurement module 97, and phrenic nerve stimulation module 98, and calculate power source longevity. Processor(s) 428 may prioritize vectors using any of the techniques described in this disclosure based on the received measurements. As mentioned above, one or more of the criteria used for prioritization may be weighted. That is, a first criterion may be less favorable than a second criterion. As such, the first criterion may be weighted more heavily than the second criterion.

The techniques described above may facilitate selection of a pacing vector by automating the measurements of one or more user-selectable prioritized criteria for each of a plurality of vectors. In addition, the prioritization techniques disclosed may also be used to provide a backup procedure for selecting another electrode or band in the case of lead failure or lead dislodgement.

## Claims

1. A system that facilitates selection of at least one vector from among a plurality of vectors for pacing a first chamber of a heart, the system comprising:
an implantable medical device (16) configured to deliver pacing stimuli to the heart by means of one of said plurality of vectors; and **characterized by**
means for receiving a selection of said plurality of vectors and measuring a plurality of criteria for each of said plurality of vectors by which each of the plurality of vectors may be prioritized;
a processor (80) configured to:
store said plurality of criteria measurements for each of the plurality of vectors;
control presentation of the plurality of criteria by which each of the plurality of vectors may be prioritized, the plurality of criteria consisting of cardiac resynchronization therapy (CRT) efficacy, capture threshold amplitude, R-wave amplitude, impedance, phrenic nerve stimulation amplitude and power source longevity;
receive a selection of at least one criterion from among the plurality of criteria by which each of the plurality of vectors may be prioritized; and
automatically prioritize the plurality of vectors based on the measurement of the at least one selected criterion; wherein the at least one selected criterion comprises power source longevity.

2. The system of claim 1 wherein the processor is further configured to:
control presentation, to the user, of at least one of the prioritized vectors.

3. The system of any of claims 1 to 2, wherein the processor is further configured to:
select at least one of the prioritized vectors for delivery of a pacing stimulus.

4. The system of any of claims 1 to 3, further comprising a user interface (314) configured to present at least one of the plurality of criteria, the at least one of the prioritized vectors, and the measurements of the at least one criterion that was not selected by the user.

5. The system of any of claims 1 to 4, further comprising a programmer (24) configured to communicate with the implantable medical device.

## Patentansprüche

1. System, das eine Auswahl von zumindest einem Vektor unter einer Mehrzahl von Vektoren zur Schrittmachtertherapie einer ersten Kammer eines Herzens erleichtert, wobei das System umfasst:
eine implantierbare medizinische Vorrichtung (16), die konfiguriert ist, um Schrittmacherstimuli an das Herz durch einen der Mehrzahl von Vektoren abzugeben; und **gekennzeichnet durch**
ein Mittel zum Empfangen einer Auswahl der Mehrzahl von Vektoren und Messen einer Mehrzahl von Kriterien für jeden der Mehrzahl von Vektoren, wodurch jeder der Mehrzahl von Vektoren priorisiert werden kann;
einen Prozessor (80), der konfiguriert ist um:
die Mehrzahl von Kriterienmessungen für jeden der Mehrzahl von Vektoren zu speichern;
eine Präsentation der Mehrzahl von Kriterien zu steuern, durch die jeder der Mehrzahl von vektoren priorisiert werden kann, wobei die Mehrzahl von Kriterien aus kardialer Resynchronisationstherapie- (CRT) Wirksamkeit, Einfangsschwellwertamplitude, R-Wellen-Amplitude, Impedanz, das Zwerchfell betreffende Nervenstimulationsamplitude, und Energiequellenlebensdauer bestehen;
eine Auswahl von zumindest einem Kriterium unter der Mehrzahl von Kriterien zu empfangen, durch die jeder der Mehrzahl von Vektoren priorisiert werden kann; und
automatisch die Mehrzahl von Vektoren basierend auf der Messung des zumindest einen Kriteriums zu priorisieren; wobei das zumindest eine ausgewählte Kriterium eine Leistungs-bzw. Energiequellenlebensdauer umfasst.

2. System nach Anspruch 1, wobei der Prozessor ferner konfiguriert ist um:
eine Präsentation gegenüber dem Nutzer von zumindest einem der priorisierten Vektoren zu steuern.

3. System nach einem der Ansprüche 1 bis 2, wobei der Prozessor ferner konfiguriert ist um:
zumindest einen der priorisierten Vektoren zum Abgeben eines Schrittmacherstumulus auszuwählen.

4. System nach einem der Ansprüche 1 bis 3, ferner umfassend eine Nutzerschnittstelle (314), die konfiguriert ist, um zumindest eins der Mehrzahl von Kriterien, den zumindest einen der priorisisierten Vektoren und die Messung von zumindest einem Kriterium, das nicht von dem Nutzer ausgewählt worden ist, zu präsentieren.

5. System nach einem der Ansprüche 1 bis 4, ferner umfassend ein Programmiergerät (24), das konfiguriert ist, um mit der implanierbaren medizinischen Vorrichtung zu kommunizieren.

## Revendications

1. Système facilitant la sélection d'au moins un vecteur parmi une pluralité de vecteurs pour stimuler une première chambre d'un coeur, le système comportant :
un dispositif médical implantable (16) configuré pour délivrer des stimuli d'entraînement au coeur au moyen d'un vecteur parmi ladite pluralité de vecteurs ; et **caractérisé par**
des moyens pour recevoir une sélection de ladite pluralité de vecteurs et mesurer une pluralité de critères pour chaque vecteur de ladite pluralité de vecteurs par l'intermédiaire desquels chaque vecteur de la pluralité de vecteurs peut être rendu prioritaire ;
un processeur (80) configuré pour :
stocker ladite pluralité de critères de mesure pour chaque vecteur de la pluralité de vecteurs ;
commander une présentation de la pluralité de critères par l'intermédiaire desquels chaque vecteur de la pluralité de vecteurs peut être rendu prioritaire, la pluralité de critères étant constituée de l'efficacité d'une thérapie de resynchronisation cardiaque (CRT), d'une amplitude de seuil de capture, d'une amplitude d'onde R, d'une impédance, d'une amplitude de stimulation du nerf phrénique et d'une longévité de source d'alimentation ;
recevoir une sélection d'au moins un critère parmi la pluralité de critères par l'intermédiaire desquels chaque vecteur de la pluralité de vecteurs peut être rendu prioritaire ; et
rendre automatiquement prioritaire la pluralité de vecteurs sur la base de la mesure du au moins un critère sélectionné ; dans lequel le au moins un critère sélectionné comporte la longévité de la source d'alimentation.

2. Système selon la revendication 1, dans lequel le processeur est en outre configuré pour :
commander une présentation, à l'utilisateur, d'au moins un des vecteurs rendus prioritaires.

3. Système selon l'une quelconque des revendications 1 à 2, dans lequel le processeur est en outre configuré pour :
sélectionner au moins un des vecteurs rendus prioritaires pour la délivrance d'un stimulus d'entraînement.

4. Système selon l'une quelconque des revendications 1 à 3, comportant en outre une interface utilisateur (314) configurée pour présenter au moins un critère parmi la pluralité de critères, le au moins un vecteur parmi les vecteurs rendus prioritaires, et les mesures du au moins un critère qui n'a pas été sélectionné par l'utilisateur.

5. Système selon l'une quelconque des revendications 1 à 4, comportant en outre un programmateur (24) configuré pour communiquer avec le dispositif médical implantable.
